(19) Europäisches Patentamt
European Patent Office
Office européen des brevets

(11) Publication number: 0 334 244 A2

# (12) EUROPEAN PATENT APPLICATION

(21) Application number: 89104872.0

(22) Date of filing: 18.03.89

(51) Int. Cl.4: C07K 7/18 , A61K 37/02 , //C07K99/18

(30) Priority: 25.03.88 US 173391
01.03.89 US 315245

(43) Date of publication of application:
27.09.89 Bulletin 89/39

(84) Designated Contracting States:
AT BE CH DE FR GB IT LI LU NL SE

(71) Applicant: THE PROCTER & GAMBLE COMPANY
One Procter & Gamble Plaza
Cincinnati Ohio 45202(US)

(72) Inventor: Gardner, Joseph Herman
4060 Boomer Road
Cincinnati, OH 45247(US)
Inventor: Correa, Paul Elliott
6511 Wesselman Road
Cincinnati, OH 45248(US)
Inventor: Berman, Elizabeth Faith
11787 Rose Lane
Cincinnati, OH 45246(US)
Inventor: Charest, Robert Paul
3935 Dust Commander Drive
Hamilton, OH 45011(US)

(74) Representative: Canonici, Jean-Jacques et al
Procter & Gamble European Technical
Center N.V. Temselaan 100
B-1820 Strombeek-Bever(BE)

## (54) Bradykinin Antagonist Peptides.

(57) The present invention involves nona- or larger bradykinin antagonist peptides where certain amino acid residues of the nonapeptide hormone bradykinin or other substituted analogs of bradykinin are altered as follows: either position 2, position 3, or both consist of a L-acidic, L-amide or L-hydroxamate amino acid residue; position 7 consists of a D-aromatic amino acid residue; and position 4 preferably consists of a L-aliphatic or a D-cyclic amino acid residue.

EP 0 334 244 A2

## BRADYKININ ANTAGONIST PEPTIDES

The present invention relates to novel peptides which act as antagonists of the biological activities of bradykinin.

### BACKGROUND OF THE INVENTION

Bradykinin, and its physiologically important related peptides kallidin (lysine-bradykinin) and methionine-lysine-bradykinin, contract smooth muscle (for example to product diarrhea and inflammatory bowel disease and asthma); lower blood pressure; mediate inflammation as in allergies, arthritis and asthma; participate in blood clotting and complement-mediated reactions in the body; mediate rhinitis (viral, allergic and nonallergic); and are overproduced in pathological conditions such as septic shock, acute pancreatitis, hereditary angioneurotic edema, post-gastrectomy dumping syndrome, carcinoid syndrome, anaphylactic shock, reduced sperm motility and certain other conditions. The production of bradykinin results in pain as the site of the pathological condition, and the overproduction of bradykinin intensifies the pain directly via stimulation by bradykinin, or by the activation of the arachidonic acid pathway which produces prostaglandins and leukotrienes and/or by release of other neuropeptides. Bradykinin and bradykinin-related kinins are not only produced by the animal, but may also be injected as a result of stings and bites. It is known that insects such as hornets and wasps inject bradykinin-related peptides which also cause pain, swelling and inflammation. Literature references describing these actions of bradykinin and related peptides are found in the Handbook of Experimental Pharmacology, Volume 25 (1970) and Volume 25 Supplement (1979), Springer-Verlag.

The existence, activity and peptide structure of bradykinin has been known since about 1960 (Boissonnas, R.A., S. Guttmann, P.A. Jaquenoud, H. Konzett, & E. Sturmer, "Synthesis and Bio logical Activity of Peptides Related to Bradykinn", Experimenta, Vol. 16 (1960), p. 326). Bradykinin is a naturally occurring agonist which binds to one or more specific biological receptors and stimulates pain and inflammatory responses and smooth muscle contraction in mammals, including man. Since elucidation of the bradykinin structure as a nonapeptide, there has been speculation that peptides with a similar structure could act as bradykinin antagonists by binding to the bradykinn receptor without stimulating the pain and inflammatory responses of tissues as bradykinin does. Several hundred such sequence-related peptide analogs have been synthesized and assayed in biological systems: Stewart, J.M., & D.W. Woolley, "The Search for Peptides with Specific Anti-Bradykinin Activity", Hypotensive Peptides, Springer-Verlag New York, Inc. (1966), pp. 23-33; Schroder, E., "Structure-Activity Relationships of Kinins", Handbook of Experimental Pharmacology, Vol. 25 (1970), Springer-Verlag, pp. 324-350; Stewart, J.M., "Chemistry and Biologic Activity of Peptides Related to Bradykinin", Handbook of Experimental Pharmacology, Vol. 25 Supplement (1979), Springer-Verlag, pp. 227-272; Stewart, J.M., & R.J. Vavrek, "Bradykinin Chemistry: Agonists and Antagonists", Advances in Experimental Medicine and Biology, Vol. 156 (1983), pp. 585-589.

U.S. Patent No. 4,693,993, issued to Stewart & Vavrek on September 15, 1987 (see also corresponding PCT Patent Application No. WO 86/07263 published December 18, 1986) discloses and claims peptides purported to be bradykinin antagonists. However, when the peptides of Stewart & Vavrek ′993 are assayed by well-known and accepted analgesic and antiinflammatory *in vivo* assay procedures (not disclosed in Stewart & Vavrek ′993), these peptides have been found to exhibit agonist activity or mixed agonist/antagonist activity.

It is an object of the present invention to provide novel peptides having activity as bradykinin antagonists.

It is a further object of the present invention to provide such bradykinin antagonist peptides having no measurable or minimal agonist activity.

It is another object of the present invention to provide novel pharmaceutical compositions containing bradykinin antagonist peptides.

It is still another object of the present invention to provide novel methods for providing analgesia and/or smooth muscle relaxation, and for treating inflammation, rhinitis, rhinorhea, sore throat pain, congestion and/or other conditions mediated by bradykinin, by administering such bradykinin antagonist peptides to humans or lower animals.

## SUMMARY OF THE INVENTION

The present invention relates to bradykinin antagonist peptides having the structure:

H-L-Q-T-U-V-W-X-Y-Z-Arg-OH (1)

wherein

(a) L is selected from the group consisting of nil, a single basic amino acid residue of the D- or L-configuration, a peptide containing from about 2 to about 4 amino acid residues of the D- or L-configuration at least one of which is basic, and an N-terminal enzyme protecting group from the group consisting of acyl-type protecting groups, aromatic urethane-type protecting groups, and alkyl-type protecting groups;

(b) Q is selected from the group consisting of Arg, Lys and Orn;

(c) T and U are each independently selected from the group consisting of D- and L-cyclic amino acid residues, D-and L-aliphatic amino acid residues, L-aromatic amino acid residues, L-acidic amino acid residues, L-amide amino acid residues, and L-hydroxamate amino acid residues; and wherein at least one of T and U is selected from the group consisting of L-acidic amino acid residues and L-amide amino acid residues and L-hydroxamate amino acid residues;

(d) V is selected from the group consisting of D- or L-aliphatic, D- or L-cyclic, and D- or L-aromatic amino acid residues;

(e) W is selected from the group consisting of L-aliphatic and L-aromatic amino acid residues;

(f) X is selected from the group consisting of D- or L-aromatic and D- or L-aliphatic amino acid residues;

(g) Y is selected from the group consisting of D-aromatic amino acid residues; and

(h) Z is selected from the group consisting of L-aromatic and L-aliphatic amino acid residues;

and the pharmaceutically acceptable salts thereof.

## DETAILED DESCRIPTION OF THE INVENTION

The present invention involves peptides which are condensations of amino acid residues in particular sequences.

For purposes of this application, amino acid residues are categorized as aliphatic, cyclic, aromatic, basic, acidic, or amide based on the primary characteristic of their functional group.

As used herein, aliphatic amino acid residues have the following chemical structure:

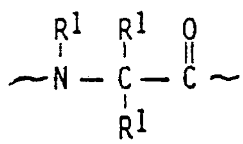

(2)

wherein each $R^1$ is independently selected from the group consisting of hydrogen and saturated or unsaturated, straight or branched chain, unsubstituted or substituted alkyl having from 1 to about 6 carbon atoms. One $R^1$ attached to the carbon may be nil such that there is a double bond between the carbon and the other $R^1$ attached to it. Substituted $R^1$ has substantially neutral (not acid or basic), non-aromatic substituents. Preferred $R^1$ are unsubstituted or substituted with hydroxy, alkoxy having from 1 to about 3 carbon atoms, halo or nitro; more preferred $R^1$ are unsubstituted or substituted with hydroxy. Preferred $R^1$ are $C_1$ to $C_4$ alkyl which are straight chain or have a single methyl branch. Preferred $R^1$ are saturated. Also preferred are aliphatic amino acid residues wherein the $R^1$ attached to the nitrogen is hydrogen and at least one of the $R^1$'s attached to the carbon is a hydrogen.

As used herein, cyclic amino acid residues have the chemical structure:

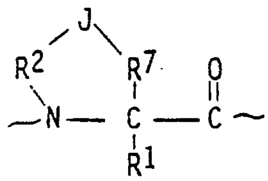

(3)

wherein $R^1$ is as defined hereinabove; wherein $R^2$ is saturated or unsaturated, straight or branched chain, unsubstituted or substituted alkyl having from about 1 to about 6 carbon atoms; wherein $R^7$ is nil or $CH_2$; and wherein J is selected from the group consisting of nil, sulfur, and oxygen. J is preferably nil or sulfur; most preferably J is nil. $R^7$ is preferably nil. $R^2$ is preferably $C_2$ to $C_4$, more preferably $C_3$ or $C_4$, most preferably $C_3$. $R^2$ is preferably saturated. $R^2$ is preferably straight chain. Substituted $R^2$ has substantially neutral, non-aromatic substituents. $R^2$ is preferably unsubstituted or substituted with hydroxy, alkoxy having from 1 to about 3 carbon atoms, halo or nitro; more preferably $R^2$ is unsubstituted or substituted with hydroxy; more preferably $R^2$ in unsubstituted. $R^1$ is preferably hydrogen, or when J is nil, $R^1$ may be nil such that there is a double bond between the carbon and $R^2$; most preferably $R^1$ is hydrogen.

As used herein, aromatic amino acid residues have the chemical structure:

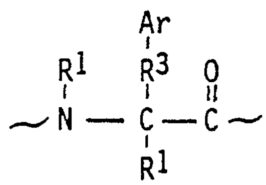

(4)

wherein each $R^1$ is as defined hereinabove; $R^3$ is selected from the group consisting of nil, methylene and ethylene; and Ar is unsub stituted or substituted aryl. Each $R^1$ is preferably hydrogen. $R^3$ is preferably methylene. Ar is preferably selected from the group consisting of unsbustituted and substituted phenyl, naphthyl, thienyl, pyridyl, indolyl and imidazolyl; most preferred Ar is unsubstituted or substituted phenyl. Substituted Ar has substantially neutral, non-aromatic substituents. Ar is preferably unsubstituted or substituted by hydroxy, $C_1$ to $C_3$ alkoxy, nitro or halo; more preferably Ar is unsubstituted or substituted with hydroxy; most preferably Ar is unsubstituted.

As used herein, basic amino acid residues have the chemical structure:

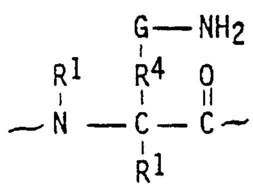

(5)

wherein each $R^1$ is as defined hereinabove; $R^4$ is saturated or unsaturated, straight or branched chain, unsubstituted alkyl having from about 1 to about 6 carbon atoms; G is selected from the group consisting of nil and -NH-C(NH)-. Each $R^1$ is preferably hydrogen. $R^4$ is preferably $C_3$ to $C_4$. $R^4$ is preferably saturated. $R^4$ is preferably straight chain.

As used herein, acidic amino acid residues have the chemical structure:

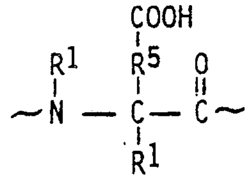 (6)

or

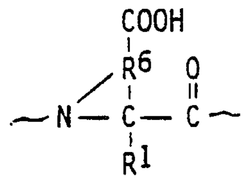 (7)

wherein each $R^1$ is as defined hereinabove; and $R^5$ and $R^6$ are saturated or unsaturated, branched or straight chain, unsub stituted alkyl having from 1 to about 6 carbon atoms. Each $R^1$ is preferably hydrogen. $R^5$ and $R^6$ are preferably $C_1$ to $C_4$; $R^5$ and $R^6$ are preferably saturated. $R^5$ and $R^6$ are preferably straight chain. $R^5$ is preferably $C_1$ to $C_2$; $R^6$ is preferably $C_3$.

As used herein, amide amino acid residues have a chemical structure the same as acidic amino acid residues as defined hereinabove, but wherein the -COOH is replaced by either $-CONH_2$ or $-NHC(O)CH_3$.

As used herein, hydroxamate amino acid residues have a chemical structure the same as amide amino acid residues as defined hereinabove having a $-CONH_2$ moiety, but wherein such $-CONH_2$ is replaced by -CONHOH.

Table 1 hereinbelow provides examples of certain amino acid residues which are a part of the peptides of the present invention, abbreviations used in this application to denote such amino acid residues, and the chemical structures of these amino acid residues.

Table 1

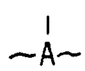 is 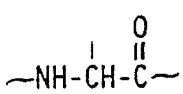 ; 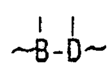 is 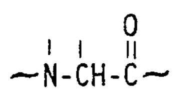

| Amino Acid Residue | Abbreviation* | Structure |
|---|---|---|
| Aliphatic | | |
| glycyl | Gly | 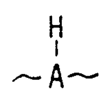 |
| alanyl | Ala | 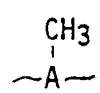 |
| valyl | Val | 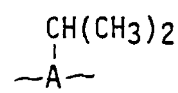 |
| leucyl | Leu | 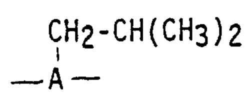 |

| Amino Acid Residue | Abbreviation* | Structure |
|---|---|---|
| Aliphatic (continued) | | |
| isoleucyl | Ile | 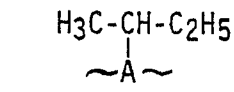 |
| seryl | Ser | 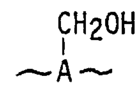 |
| threonyl | Thr | 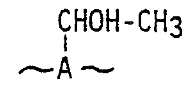 |
| α-aminoisobutyryl | Aib | 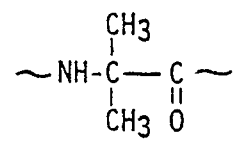 |
| sarcosyl | Sar | 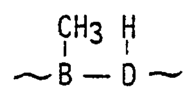 |
| Cyclic | | |
| prolyl | Pro | 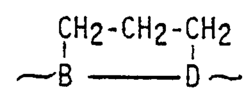 |
| hydroxyprolyl | Hyp | 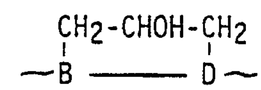 |
| 2-carboxy-thiazolidyl | Thz | 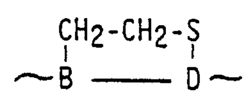 |
| 2-carboxy-azetidyl | Azt | 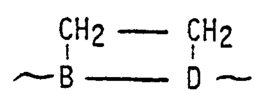 |
| pipecolinyl | Pip | 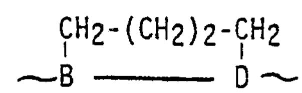 |
| 2,3-dehydroprolyl | Epr | 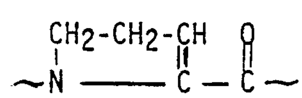 |

| Amino Acid Residue | Abbreviation* | Structure |
|---|---|---|
| Aromatic | | |
| phenylalanyl | Phe | 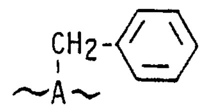 |
| tyrosyl | Tyr | 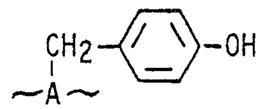 |
| β-(2-thienyl)alanyl | Thi | 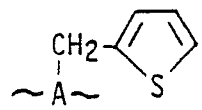 |
| β-(1-naphthyl)alanyl | 1Nap** | 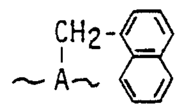 |
| β-(2-naphthl)alanyl | 2Nap** | 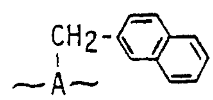 |
| β-(2-pyridyl)alanyl | Pyr | 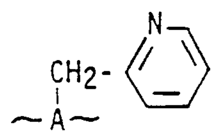 |
| p-nitrophenylalanyl | Nph | 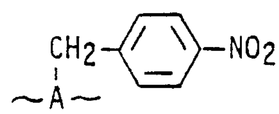 |
| p-chlorophenylalanyl | Cph | 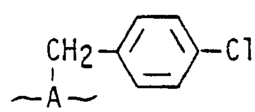 |
| O-methyltyrosyl | Mty | 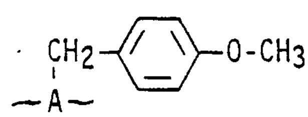 |
| tryptophyl | Trp | 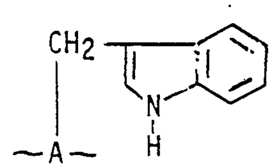 |

| Amino Acid Residue | Abbreviation* | Structure |
|---|---|---|
| Aromatic (continued) | | |
| histidyl | His | 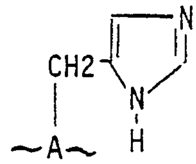 |
| homophenylalanyl | Hph | 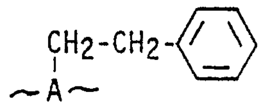 |
| α-phenylglycyl | Pgl | 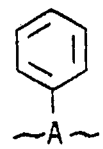 |
| Basic | | |
| arginyl | Arg | 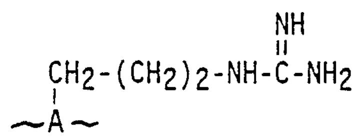 |
| homoarginyl | Har | 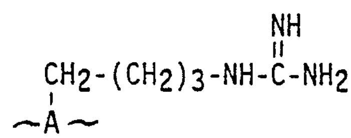 |
| lysyl | Lys | 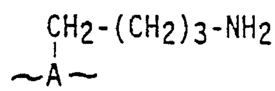 |
| ornithyl | Orn | 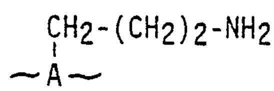 |
| Acidic | | |
| aspartyl | Asp | 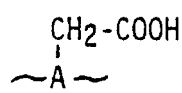 |
| glutamyl | Glu | 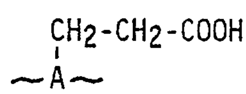 |

| Amino Acid Residue | Abbreviation* | Structure |
|---|---|---|
| Acidic (continued) | | |
| 4-carboxyprolyl | Cpr | 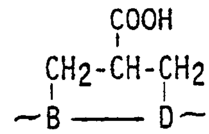 |
| Amide | | |
| asparaginyl | Asn | 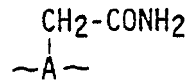 |
| glutaminyl | Gln | 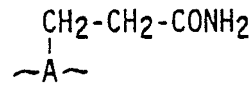 |
| 4-carbamoylprolyl | Cmp | 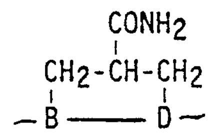 |
| N-acetyl-4-aminoprolyl | Aap | 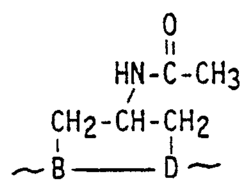 |
| N-acetylornithyl | Aor | 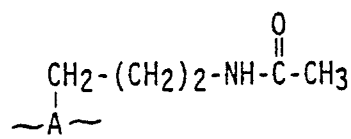 |
| Hydroxamate | | |
| N-hydroxyasparaginyl | Nha | 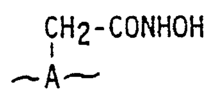 |
| N-hydroxyglutaminyl | Nhg | 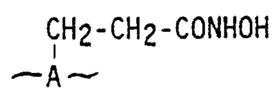 |

-----------

*Any three-letter abbreviation as provided in this Table 1 refers to an amino acid residue of the L-configuration, (except for

glycyl). An amino acid residue of the D-configuration is denoted by the same three-letter abbreviation preceded by the letter "D".
**Collectively Nap

The present invention involves bradykinin antagonist nona-(or larger) peptides having the structure:
H-L-Q-T-U-V-W-X-Y-Z-Arg-OH (1)
Position: 0 1 2 3 4 5 6 7 8 9

In Structure (1), L is selected from the group consisting of nil; a single basic amino acid residue of the D- or L-configuration; a peptide containing from 2 to about 4 amino acid residues of the D- or L-configuration, at least one of which is a basic amino acid residue; and an N-terminal enzyme protecting group from the group consisting of acyl-type protecting groups, aromatic urethane-type protecting groups, and alkyl-type protecting groups such as, for example, acetyl, t-butyloxycarbonyl (Boc), and benzyloxycarbonyl. Preferred examples of L include Arg, DArg, Lys, DLys, Lys-Lys, Met-Lys, Gly-Arg-Met-Lys, DLys-Lys, Orn, Har, DHar, 5-guanidinopentanoyl, 6-guanidinohexanoyl, 6-aminohexanoyl, and 5-aminopentanoyl. More preferred L is selected from the group consisting of DArg, Lys-Lys and nil; most preferred L is Lys-Lys and especially DArg.

In structure (1), Q is selected from the group consisting of Arg, Lys and Orn. Most preferred Q is Arg.

In Structure (1), T and U are each independently selected from the group consisting of D- and L-cyclic amino acid residues, D- and L-aliphatic amino acid residues, L-aromatic amino acid residues, L-acidic amino acid residues, L-amide amino acid residues, and L-hydroxamate amino acid residues. In addition, at least one of T and U is selected from the group consisting of L-acidic amino acid residues, L-amide amino acid residues and L-hydroxamate amino acid residues. Preferred examples of T and U include Pro, DPro, Hyp, Azt, Thz, Inp, Epr, Asp, Glu, Asn, Gln, Cpr, Cmp, Aap, Aor, Nha and Nhg. It is preferred that one of T or U (preferably T) is selected from the group consisting of D- and L-cyclic amino acid residues, D- and L-aliphatic amino acid residues and L-aromatic amino acid residues, more preferably from Pro, DPro, Hyp, Azt, Thz, Inp and Epr, more preferably still from Pro and Hyp, most preferably Pro; while the other of T or U (preferably U) is selected from the group consisting of L-acidic amino acid residues, L-amide amino acid residues and L-hydroxamate amino acid residues, more preferably from Asp, Glu, Asn, Gln, Cpr, Cmp, Aap, Aor, Nha and Nhg, more preferably still from Asn, Cmp, Aap, Gln, Aor, Nha and Nhg; most preferably from Asn and Gln.

In Structure (1), V is selected from the group consisting of aliphatic, cyclic and aromatic amino acid residues of the D- or L-configuration. Preferred V is selected from the group consisting of Gly, Ala, DPro, DHyp and Sar; more preferred V are Gly and DPro; most preferred V is Gly.

In Structure (1), W is selected from the group consisting of aliphatic and aromatic amino acid residues of the L-configuration, preferably L-aromatic amino acid residues. Preferred examples of W include Phe, Thi, Nap, Pyr, Nph, Cph, Mty and Tyr. More preferred W is selected from the group consisting of Phe and Tyr; most preferred W is Phe.

In Structure (1), X is selected from the group consisting of aromatic and aliphatic amino acid residues of the D- or L-configuration. Preferred examples of X include Ser, Thr, Ala, Gly, DPhe, DThi, DNap, DPyr and Dcph. More preferred X is selected from the group consisting of Ser, Thr, Ala and Gly; most preferred X is Ser.

In Structure (1), Y is selected from the group consisting of aromatic amino acid residues of the D-configuration. Preferred examples of Y include DPhe, DTyr, DThi, DNap, DPyr, DNph, DCph, DMty, DTrp, DHis, DHph and DPgl; more preferred Y is selected from the group consisting of DPhe, DTyr, DTrp and DHis; most preferred Y is DPhe.

In Structure (1), Z is selected from the group consisting of aromatic and aliphatic amino acid residues of the L-configuration. Preferred examples of Z include Phe, Tyr, Pro, Thi, Pyr, Nap, Mty, Nph and Cph. More preferred Z is selected from the group consisting of Phe and Tyr; most preferred Z is Phe.

The synthesis of the peptides of Structure (1), including derivatization, activation, and coupling of protected amino acid residues, and their purification, and the analytical methods for determining the identity and purity are included in the general body of knowledge of peptide chemistry, as described, for example, in Kisfaludy, L., "Repetitive Methods in Solution", Chapter 6 of The Peptides, Vol. 2, Academic Press (pub.), 1980, for solution phase synthesis, and in Stewart, J.M. & J.D. Young, Solid Phase Peptide Synthesis, Freeman (pub.), 1969, for synthesis by the solid-phase method of Merrifield. A chemist skilled in

the art of peptide synthesis can synthesize the peptides of Structure (1) by standard solution methods or by manual or automated solid-phase methods.

## EXAMPLES OF PEPTIDE SYNTHESIS

The following non-limiting examples further describe the peptides of the present invention and methods of making them.

### Example 1

### Preparation of H-Arg-Pro-Asn-Gly-Phe-Ser-DPhe-Phe-Arg-OH ($Asn^3$, $DPhe^7$-bradykinin)

One gram of Boc-Arg(Tos)-polystyrene resin (Bachem Inc., Torrence, California) (Tos-tosyl) is loaded into the reaction vessel of a Biosearch 9500 automated peptide synthesizer. The peptide synthesis program is initiated by subjecting the amino acid-resin (0.30 mmol/g) to the Boc removal sequence as described below.

#### Boc Removal Sequence:

The amino acid-resin (or peptide-resin) is washed five times with methylene chloride (20 mL, 15 seconds per wash). The Boc group is removed by two successive treatments with 45% trifluoroacetic acid, 2.5% anisole in methylene chloride (60 seconds per treatment). The deprotected resin is then washed 2 times with methylene chloride and 3 times with a 1:1 mixture of methylene chloride:dimethylformamide and entered into the neutralization sequence.

#### Neutralization Sequence:

The trifluoroacetic salt of the amino acid-resin is washed two times with methylene chloride followed by three treatments with 10% diisopropylethylamine in methylene chloride. The amino acid resin containing the free amino group is washed 5 times with methylene chloride and then is ready for the coupling sequence.

#### Coupling Sequence:

The amino acid resin is washed once with a 2:1 mixture of methylene chloride:dimethylformamide. The coupling reaction is initiated with the addition of a 1:1 mixture of 0.6 M diisopropylcarbodiimide (Aldrich Chemical Company, Milwaukee, Wisconsin): 0.6 M Boc-L-phenylalanine (Bachem, Inc.) in methylene chloride (in 6.67 mole excess). The coupling reaction is mixed with bubbling nitrogen for 1 hour. After the reagents are removed by filtration the resin is washed successively with 1:1 methylene chloride:dimethylformamide (2X), methylene chloride (2X), 10% diidopropylethylamine (1X), methylene chloride (5X), and 1:1 methylene chloride:dimethylformamide. The resin is then recoupled for 1 hour (6.67 mole excess) with a 1:1 mixture of Boc-L-phenylalanine:diispropylcarbodiimide. After removal of excess reagents the dipeptide-resin is washed twice with a 1:1 mixture of methylene chloride:dimethylformamide.

The Boc-Phe-Arg(Tos)-resin is then treated with the Boc-removal sequence described above followed by the neutralization sequence described above. The free amine-dipeptide-resin is then coupled with Boc-D-phenylalanine according to the coupling sequence described above to form the Boc-DPhe-Phe-Arg(Tos)-resin (tripeptide-resin).

The remaining amino acids are added in the following order: Boc-Ser, Boc-Phe, Boc-Gly, Boc-Asn, Boc-Pro, Boc-Arg(Tos) using the same Boc removal, neutralization and coupling sequences described above with the following exceptions: 1.5 equivalents of 1-hydroxybenzotriazole (Aldrich Chemical Company) are

added during the Asn and Arg couplings to supress unwanted side reactions known to occur when coupling these residues.

After the nonapeptide (nine amino acid peptide) is assembled on the resin as described above the peptide is cleaved from the polystyrene resin using the following HF (hydrofluoric acid) reaction.

HF Cleavage Reaction:

The completed peptide-resin (2.3 gm) is suspended in 40 mL of condensed hydrofluoric acid containing 2 mL of anisole at -78° C. The reaction is stirred for 1 hour at -78° C followed by 1 hour at 0° C. The liquid HF is removed at 0° C under reduced pressure with a slow bleed of anhydrous argon gas passing through the reaction vessel. The free peptide is extracted from the resin by washing the resin-peptide mixture with 2N acetic acid. The acetic acid washes are combined and concentrated under vacuum to yield the crude peptide product.

The peptide is purified by first passing the crude peptide through a G-10 sephadex column (Sigma Chemical Company, St. Louis, Missouri), eluting with 1N acetic acid. Appropriate fractions are pooled after analysis by thin layer chromatography and then lyophilized to yield a white amorphous solid. The peptide is further purified using reverse phase chromatography on a $C_{18}$ silica column (2.14 X 30 cm) (Rainin Instruments, Inc., Wolburn, Massachusetts), eluting with a 30 minute gradient of 10-40% solvent B mixed with solvent A (solvent A = 0.1% trifluoroacetic acid in water, solvent B = 0.1% trifluoroacetic acid in acetonitrile). The appropriate fractions are pooled and lyophilized to yield the purified peptide product.

The peptide is characterized by mass spectral analysis on a Finnigan model TSQ-46C triple quadrapole instrument using the fast atom bombardment ionization technique from a glycerol matrix. The observed molecular ion, M+H = 1127, agrees with the calculated molecular weight for the desired structure. The synthetic peptide is also sequenced using Edman degradation sequencing techniques on an automated Applied Biosystems #420 sequenator to confirm the structure of the synthetic peptide.

## Example 2

### Preparation of H-Arg-Pro-Gln-Gly-Phe-Ser-DPhe-Phe-Arg-OH $Gln^3$, $DPhe^7$-bradykinin)

The peptide is made using the same methods described in Example 1 with the replacement of Gln for Asn in the coupling reaction for position 3 in the peptide sequence. The final peptide is analyzed by mass spectral analysis as described in Example 1 giving the correct molecular ion, M+H = 1141.

## Example 3

### Preparation of H-Arg-Pro-Asn-DPro-Phe-Ser-DPhe-Phe-Arg-OH ($Asn^3$, $DPro^4$, $DPhe^7$-bradykinin)

The peptide is made using the same methods described in Example 1 with the replacement of DPro for Gly in the coupling reaction for position 4 in the peptide sequence. The final peptide is characterized by mass spectral analysis as described in Example 1 giving the correct molecular ion, M+H = 1167.

## Example 4

### Preparation of H-Arg-Asn-Pro-Gly-Phe-Ser-DPhe-Phe-Arg-OH ($Asn^2$, $DPhe^7$-bradykinin

The peptide is made using the same methods described in Example 1 with the replacement of Asn for Pro and Pro for Asn in the coupling reaction for positions 2 and 3, respectively, in the peptide sequence. The final peptide is characterized by mass spectral analysis as described in Example 1 giving the correct molecular ion, M+H = 1127.

## Example 5

### Preparation of H-Arg-Pro-Asp-Gly-Phe-Ser-DPhe-Phe-Arg-OH ($Asp^3$, $DPhe^7$-bradykinin):

The peptide is made using the same methods described in Example 1 with the replacement of Asp for Asn in the coupling reaction for position 3 in the peptide sequence. The final peptide is characterized by mass spectral analysis as described in Example 1 giving the correct molecular ion, M+H = 1128.

## Example 6

### Preparation of H-Arg-Pro-Aap-Gly-Phe-Ser-DPhe-Phe-Arg-OH ($Aap^3$, $DPhe^7$-bradykinin) and the preparation of the amino acid Aap)

Prior to the automated synthesis of the nonapeptide, the precursor of the unnatural amino acid residue N-acetyl-4-aminoprolyl (Aap) is prepared using the following procedure.

N-Boc-4-hydroxyproline benzyl ester:

N-tert-butyloxycarbonyl-4-hydroxyproline (Boc-Hyp) (Bachem, Inc.), is converted to its benzyl ester as follows. Boc-Hyp (20 g, 86 mmol) is dissolved in dimethylformamide (500 mL); potassium carbonate (5 equivalents) is added. The mixture is stirred vigorously while adding benzyl bromide dropwise over a period of 15 minutes. The reaction is heated to 55° C and stirred for 1.5 hours at that temperature. After cooling to room temperature the action mixture is diluted with 1.5 L of ethyl acetate and placed in a separatory funnel. The ethyl acetate layer is washed sequentially with water (2 X 1.2 L), 0.5 N hydrochloric acid (1 L), water (1.5 L), and saturated sodium chloride (1.5 L). The organic layer is dried over magnesium sulfate, filtered and evaporated to dryness. The resulting crude product is purified using chromatography on silica gel column with 70:30 hexane: acetone as eluant. The appropriate fractions are pooled after TLC analysis yielding 22.8 g of clear viscous liquid (82%).

N-Boc-4-azidoproline benzyl ester:

The N-Boc-4-hydroxyproline (10 g, 31.2 mmole) from above is dissolved in dimethylformamide (14 mL), and triethylamine (10.7 mL) is added. The solution is chilled to 0° C and methane sulfonyl chloride (mesyl chloride 4.1 mL) is added dropwise over a period of 12 minutes. The reaction is stirred at 0° C for 1 hour followed by 1 hour at room temperature. The intermediate 4-O-mesyl proline is not isolated but is converted directly to the azide with the addition of sodium azide (15.2 g) into the above reaction mixture followed by stirring for 20 hours at room temperature. After the first 20 hours, an additional 15.2 g of sodium azide is added and the reaction is stirred for an additional 72 hours at room temperature. The reaction mixture is transferred to a separatory funnel and diluted with 1 L of water. The aqueous mixture is extracted twice with ethyl acetate (2 X 1 L) and the ethyl acetate extracts are combined and washed with water (1 L), 0.5 N HCl (1 L), water (1 L), saturated sodium chloride (1 L). The ethyl acetate layer is dried over magnesium sulfate and evaporated to dryness. The resulting crude product is purified on silica gel chromatography eluting with 8:2 hexane:acetone. The appropriate fractions are pooled after TLC analysis yielding 3.4 g of product

(showing a strong band in IR at 2100 $cm^{-1}$).

N-Boc-4-aminoproline:

The N-Boc-4-azidoproline benzyl ester from above (3.4 g, 9.8 mmol) is dissolved in methanol and placed under an inert atmosphere. Palladium on carbon (380 mg, 10%) is added and the reaction vessel is placed in a Parr hydrogenation apparatus and pressurized with 40 psi of hydrogen gas. The reaction vessel is shaken vigorously for 3 hours and then removed from the Parr apparatus. The resulting mixture is filtered through celite and the methanol is removed in vacuo. The resulting while solid product is used directly in the following acetylation reaction.

α-N-Boc-N-(acetyl)-4-aminoproline:

The N-Boc-4-aminoproline from above (1.4 g, 6.1 mmol) is dissolved in 9 mL of 2.5 N NaOH, and 9 mL of p-dioxane is added. The resulting mixture is chilled in an ice bath to about 10° C, and acetic anhydride (1.0 mL) is added dropwise over a period of 10 minutes. The reaction is stirred for 30 minutes at 0° C, followed by stirring for 1 hour at room temperature. The reaction mixture is diluted with water (200 mL) and extracted twice with ethyl ether. The aqueous layer is acidified to pH 2-3 with the addition of solid potassium hydrogen sulfate, then extracted with ethyl acetate (2 X 200 mL). The ethyl acetate extracts are combined, washed with saturated sodium chloride, dried over magnesium sulfate, and evaporated to dryness. The final product is analyzed by $^{1}H$- and $^{13}C$-NMR, mass spec. M+ = 272, providing the correct structure.

The N-acetyl-4-aminoproline is incorporated into position 3 of the peptide sequence indicated in this example above using the same methods of peptide synthesis described in Example 1. The structure of the nonapeptide is confirmed by mass spectral analysis, M+H = 1167, and amino acid sequencing as described in Example 1.

Example 7

Preparation of H-Arg-Pro-Cpr-Gly-Phe-Ser-DPhe-Phe-Arg-OH ($Cpr^3$, $DPhe^7$-bradykinin) and the preparation of the amino acid Cpr

Prior to the automated synthesis of the nonapeptide, the precursor for the unnatural amino acid residue 4-carboxyprolyl (Cpr) is prepared using the following procedure.

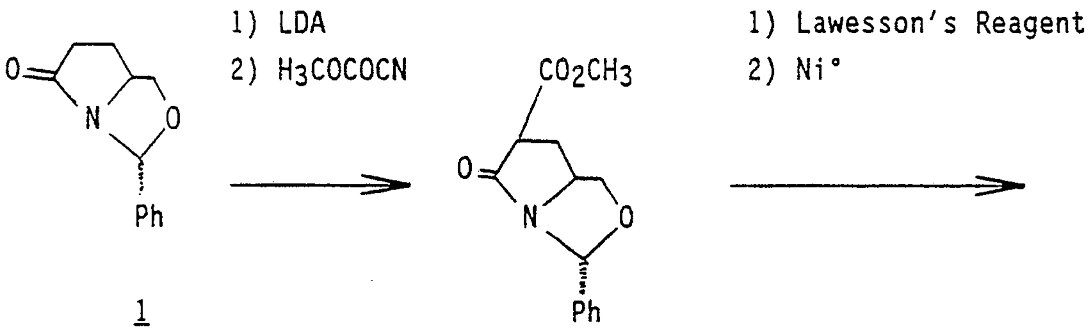


1

2

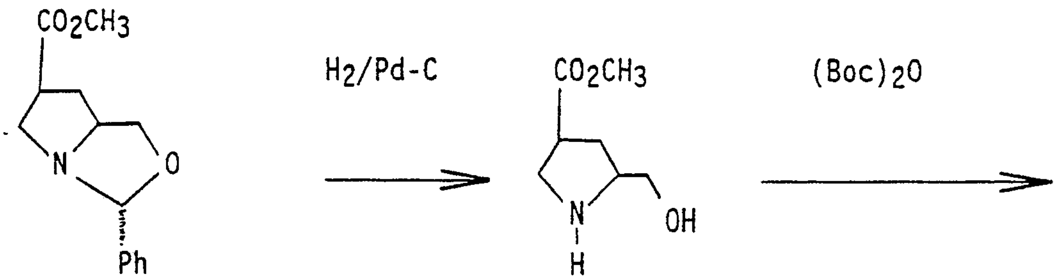


3

4

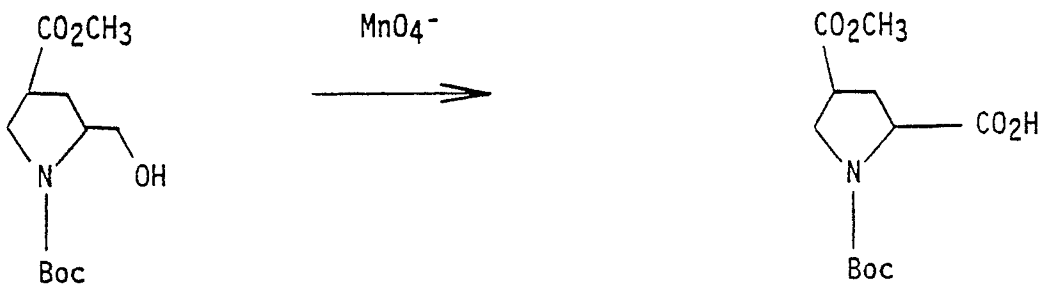


5

6

Methoxycarbonyl derivative (2):

The O,N-acetal (1[1]) is added to a solution of lithium diisopropylamide in THF (prepared in the usual way from diisopropylamine and n-butyllithium) at -78 °C with vigorous stirring. After stirring another 30 minutes at -78 °C, a slight molar excess of methyl cyanoformate is added, and the temperature is raised to -15 °C with stirring for another hour. Quenching and purification give Compound 2.

([1]Thottathil, J.K., J.L. Moniot, R.H. Mueller, M.K.Y. Wong, T.P. Kissick, J. Org. Chem., Vol. 51, (1986) p. 3140.)

Protected prolinol derivative (3):

Compound 2 is treated with Lawesson's Reagent in warm THF to give the corresponding thiolactam. This is treated with Raney nickle in refluxing ethanol until reduction is complete. Purification gives Compound 3.

4-Methoxycarbonyl-L-prolinol (4):

Compound 3 is subjected to hydrogenolysis in methanol over 10% Pd on carbon at 45 psi until the starting material is consumed. Purification gives Compound 4.

N-Boc-4-methoxycarbonyl-L-prolinol (5):

Compound 4 is treated with $(Boc)_2O$ in methanol with triethylamine at room temperature overnight. Purification gives compound 5.

N-Boc-4-Methoxycarbonyl-L-proline (6):

Compound 5 is oxidized with mildly alkyline permanganate in aqueous acetone. Purification gives Compound 6.

Compound 6 is incorporated into position 3 of the peptide sequence indicated in this example above using the same methods of peptide synthesis described in Example 1. The methyl ester of Compound 6 is cleaved by the final hydrogen fluoride cleavage of the peptide from the resin resulting in the amino acid residue Cpr in position 3 as shown. The structure of the nonapeptide is confirmed by mass spectral analysis, M + H = 1154, and the amino acid sequencing as described in Example 1.

## Example 8

### Preparation of H-Arg-Pro-Cmp-Gly-Phe-Ser-DPhe-Phe-Arg-OH ($Cmp^3$, $DPhe^7$-bradykinin) and the preparation of the amino acid Cmp)

Prior to automated synthesis of the monapeptide, the precursor for the unnatural amino acid residue 4-carbomoxylprolyl (Cmp) is prepared as follows.

N-Boc-4-carbamoyl-L-proline (Boc-Cmp) (7):

Compound 6 from Example 7 is treated with methanol saturated with ammonia. Purification gives Compound 7.

Compound 7 is incorporated into position 3 of the nonapeptide sequence indicated in this example above using the same methods of peptide synthesis described in Example 1. The structure of the nonapeptide is confirmed by mass spectral analysis, M+H = 1153, and the amino acid sequencing as described in Example 1.

## Example 9

### Preparation of H-Arg-Pro-Cmp-Gly-Phe-Ser-DPhe-Phe-Arg-OH ($Cmp^3$,$DPhe^7$-bradykinin) and the preparation of the amino acid Cmp

Prior to the automated synthesis of the nonapeptide, the precursor for the unnatural amino acid residue 4 carboxamidoproline (Cmp) is prepared using the following procedure.

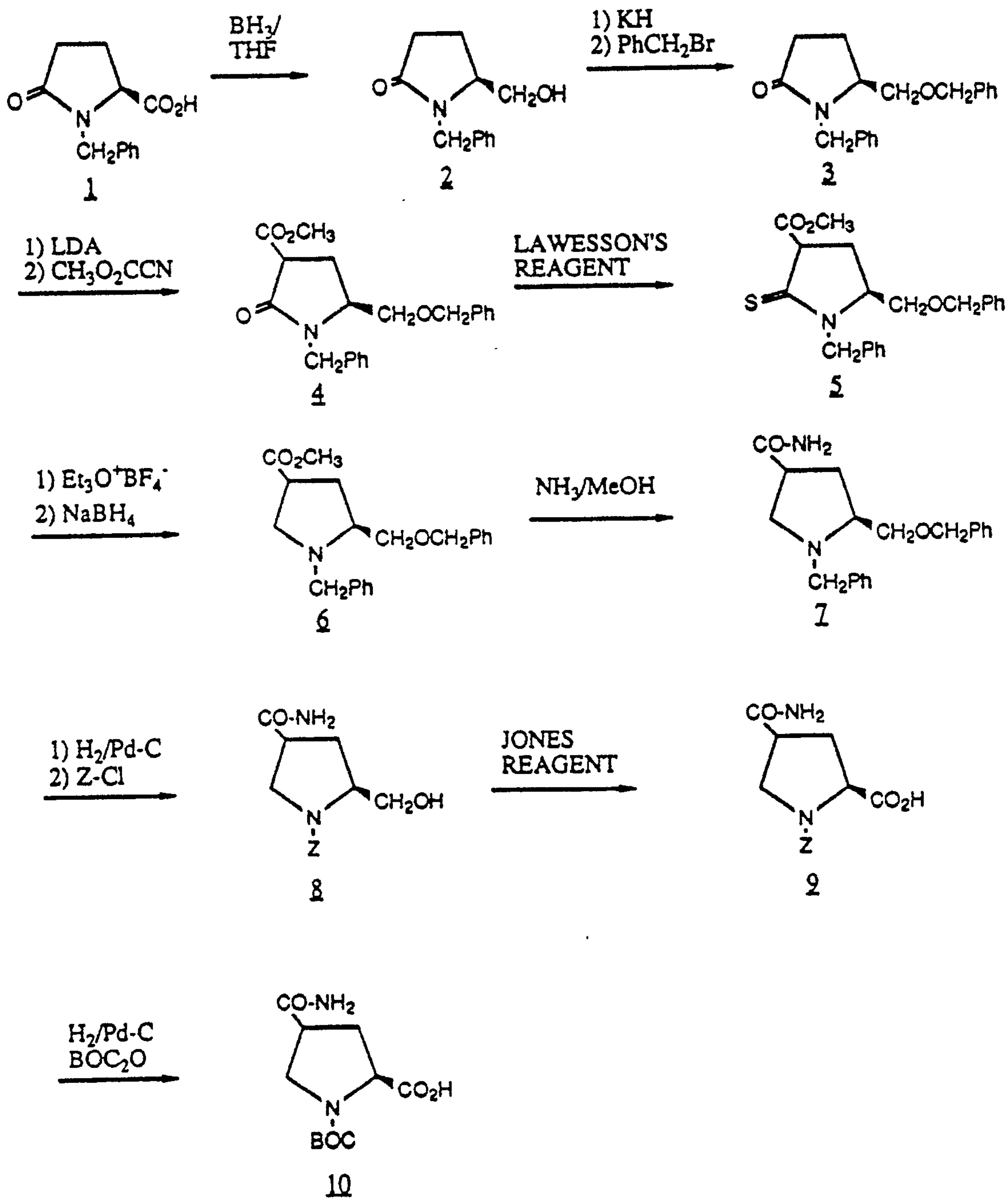


1 2 3 4 5 6 7 8 9 10

N-Benzyl pyroglutaminol (2):

A solution of borane in THF (1.4 equivalents) is added dropwise to a stirred solution of N-benzyl-L-pyroglutamic acid (1; See Petersen, J.S., G. Fels & H. Rapoport, Journal of the American Chemical Society, Vol. 106, (1984), p. 4539) in THF at 0° C, under argon. The solution is then stirred at room temperature overnight. The reaction is quenched by adding saturated aqueous $K_2CO_3$, then extracted with ethyl acetate. The extracts are dried and concentrated, and the residue is purified by chromatography (silica gel; $CHCl_3$/MeOH - 97/3). The product is crystallized from EtOAc/Hexane and has mp 83-84° C.

N-Benzyl pyroglutaminol benzyl ether (3):

A solution of Compound 2 in THF is added to a slight excess of potassium hydride suspended in THF at 0 ° C, under argon. After stirring for 1 hour, benzyl bromide is added, and the solution is warmed to room temperature and stirred another hour. The reaction is quenched with aqueous $NaHCO_3$, and extracted with ether. The extracts are dried and concentrated, and the residue is purified by chromatography (silica gel; $CH_2Cl_2$/EtOAC - 90/10). Compound 3 is obtained as an oil.

Methoxycarbonyl derivative (4):

Compound 3 is dissolved in THF and added to a solution of lithium diisopropylamine (2 equivalents) in THF (prepared in the usual way from diisopropylamine and n-butyllithium) at -78 ° C. The solution is warmed to 30 ° C over one hour, then cooled again to -78 ° C when methyl cyanoformate (1.05 equivalents) is added. After 10 minutes the solution is poured into 5% aqueous citric acid and extracted with EtOAc. The extracts are dried and concentrated and the residue is purified by chromatography to give Compound 4 as an oil.

Thiolactam (5):

Compound 4 is treated with Lawesson's Reagent in toluene at 60 ° C for 5 hours. Chromatography gives Compound 5 as an oil.

Protected prolinol derivative (6):

Compound 5 is treated with triethyloxonium tetrafluoroborate (1.1 equivalents) in $CH_2Cl_2$ for 1 hour at room temperature. The solvent is evaporated and replaced with methanol. Sodium boro hydride (2 equivalents) is added, and the mixture is stirred for 1 hour. Purification provides Compound 6.

Carboxamide derivative (7):

Compound 6 is dissolved in ammonia saturated methanol and stirred for 3 days. Evaporation gives pure Compound 7.

Carbobenzoxy derivative (8):

Compound 7 is dissolved in glacial acetic acid treated with 10% palladium on carbon, and shaken under hydrogen (45 psi) until both benzyl groups are removed. The catalyst is removed by filtration and the solvent is evaporated. The residue is dissolved in water containing NaOH (3 equivalents) and treated with benzylchloroformate. The mixture is stirred vigorously for 4 hours, then extracted with EtOAc. The extracts are dried and concentrated, and the residue is purified by chromatography to give Compound 8.

N-Z-4-carboxamidoproline (9):

Compound 8 is dissolved in acetone and added to a stirred solution of Jones reagent in acetone at 0 ° C. After stirring several hours, isopropyl alcohol is added and stirring is continued another 20 minutes. After removing the organic solvents, the residue is diluted with water and extracted with EtOAc. The extracts are dried and concentrated to give Compound 9.

N-Boc-4-carboxyamidoproline (10):

The Z-group of Compound 9 is removed by hydrogenation in methanol with palladium/carbon catalyst. The N-Boc group is then introduced by stirring the secondary amine with $Boc_2O$ and triethylamine in

aqueous THF. Reverse phase chromatography is required to provide pure Compound 10.

Compound 10 is incorporated into position 3 of the peptide sequence indicated above using the same methods of peptide synthesis described in Example 1. Hydrogen fluoride cleavage and purification as described above gives the nonapeptide. The structure of the nonapeptide is confirmed by mass spectral analysis, M + H = 1153 and the amino acid sequencing as described in Example 1.

Example 10

Preparation of H-Arg-Pro-Amp-Gly-Phe-Ser-DPhe-Phe-Arg-OH ($Amp^3$,$DPhe^{76}$-bradykinin) and the preparation of α-N-Boc-N-Z-4-aminoproline

Prior to the automated synthesis of the nonapeptide, the precursor for the unnatural amino acid residue 4-aminoproline (Amp) is prepared using the following procedure.

α-N-Boc-N-Z-4-aminoproline:

N-Boc-4-aminoproline (see Example 6) is dissolved in a 1:1 mixture of dioxane and 2.5N NaOH. After chilling on ice, benzylchloroformate is added, and the mixture is stirred 30 minutes on ice, then 1 hour at room temperature. The mixture is diluted with water, washed with ether and then acidified to pH 2. Extraction with EtOAc and removal of solvent gives α-N-Boc-N-Z-4-aminoproline as a white solid.

The α-N-Boc-N-Z-4-aminoproline is incorporated into position 3 of the peptide sequence indicated above using the same methods of peptide synthesis described in Example 1. The Z-group is removed by the final hydrogen fluoride cleavage of the peptide from the resin resulting in the amino acid residue Amp in position 3 as shown. The structure of the nonapeptide is confirmed by mass spectral analysis, M + H = 1125, and amino acid sequencing as described in Example 1.

Example 11

Preparation of H-Arg-Pro-Aor-Gly-Phe-Ser-DPhe-Phe-Arg-OH ($Aor^3$, $DPhe^7$-bradykinin)

Prior to the automated synthesis of the nonapeptide, the precursor for the amino acid residue Aor is prepared using the following procedure:

N(5)-Acetyl-N(2)-Boc-Ornithine (N-Boc-Aor):

To a solution of N(2)-Boc-ornithine (Bachem, Inc.) (2 mmol) and sodium carbonate (4 mmol) in water (10 mL) is added acetic anhydride (2.5 mmol). This mixture is stirred at room temperature for 3 hours, then acidified with solid citric acid and extracted several times with chloroform. Drying and removal of volatiles gives N-Boc-Aor.

The peptide is made using the same methods described in Example 1 with the replacement of N-Boc-Aor in the coupling reaction for position 3 of the peptide sequence. Cleavage and purification are accomplished as in Example 1 with the exception that the gradient for reverse phase chromatography is 20-35% solvent B (See Example 1). The purified peptide is analyzed by mass spectral analysis as described in Example 1 giving the correct molecular ion, M + H = 1169, and amino acid sequencing as described in Example 1.

Example 12

Preparation of H-DArg-Arg-Pro-Asn-Gly-Phe-Ser-DPhe-Arg-OH ($DArg^0$, $Asn^3$, $DPhe^7$-bradykinin)

The peptide is made using the same methods described in Example 1 except that an additional residue of Boc-DArg(Tos) is added at the N-terminal end of the peptide following the Boc removal sequence described in Example 1. The final peptide is analyzed by mass spectral analysis as described in Example 1 giving the correct molecular ion, M + H = 1283, and amino acid sequencing as described in Example 1.

Example 13

Preparation of H-DArg-Arg-Pro-Aap-Gly-Phe-Ser-DPhe-Phe-Arg-OH ($DArg^0$, $Aap^3$, $DPhe^7$-bradykinin

The peptide is made using the same methods described in Example 1. The sequence is identical to that in Example 6 except that an additional residue of DArg is added to the N-terminus by coupling Boc-DArg(Tos) to the N-terminal end of the peptide following the Boc removal sequence described in Example 1. The final peptide is analyzed by mass spectral analysis as described in Example 1 giving the correct molecular ion, M + H - 1323, and amino acid sequencing as described in Example 1.

Example 14

Preparation of H-DArg-Arg-Pro-Cmp-Gly-Phe-Ser-DPhe-Phe-Arg-OH ($DArg^0$, $Cmp^3$, $DPhe^7$-bradykinin)

The peptide is made using the same methods described in Example 1. The sequence is identical to that in Example 7 except that an additional residue of DArg is added to the N-terminus by coupling Boc-DArg(Tos) to the N-terminal end of the peptide following the Boc removal sequence described in Example 1. The final peptide is analyzed by mass spectral analysis as described in Example 1 giving the correct molecular ion, M + H = 1309, and amino acid sequencing as described in Example 1.

Example 15

Preparation of H-DArg-Arg-Pro-Nha-Gly-Phe-Ser-DPhe-Phe-Arg-OH ($DArg^0$, $Nha^3$, $DPhe^7$-bradykinin)

The peptide is made using the same methods described in Example 1. N(4)-Hydroxyaspargine (Nha) is incorporated into the peptide by using N-Boc-aspartic acid $\beta$-methyl ester in the coupling reaction at position 3. DArg is incorporated into position 0 using Boc-DArg(Tos). The hydrogen fluoride cleavage leaves the methyl ester intact. After cleavage, the peptide is treated with hydroxylamine in aqueous solution to form the Nha residue. Purification is then accomplished as described in Example 1. The final peptide is analyzed by mass spectral analysis as described in Example 1 giving the correct molecular ion, M + H = 1299, and amino acid sequencing as described in Example 1.

Example 16

## Preparation of H-Lys-Lys-Arg-Pro-Asn-Gly-Phe-Ser-DPhe-Phe-Arg-OH (Lys-$Lys^0$, $Asn^3$, $DPhe^7$-bradykinin)

The peptide is made using the same methods described in Example 1 and using the same sequence except after the $Arg^1$ is coupled the Boc removal sequence is again employed. Then N(2)-Boc-N(6)-CBZ-lysine is coupled, the peptide is cleaved as described in Example 1 and HPLC purification gives the peptide. The final peptide is analyzed by mass spectral analysis as described in Example 1 giving the correct molecular ion, M + H = 1383, and amino acid sequencing as described in Example 1.

## METHODS FOR DETERMINING BRADYKININ ANTAGONIST ACTIVITY

The following test methods describe procedures used to measure the bradykinin antagonist activity of the peptides of the present invention. Different assays are considered predictive of the activity of the peptides on different tissues. A peptide which is an antagonist in one assay may be an agonist in other assays. Preferred bradykinn antagonist peptides are antagonists in all the predictive assays.

### Test Method 1

### Guinea Pig Ileum Assay

The bradykinin antagonists are assayed on guinea pig ileum for inhibition of the muscle-contracting activity of bradykinin, according to the commonly accepted assay method for bradykinin and related kinins as described in Stewart, J.M., "Chemistry and Biological Activity of Peptides Related to Bradykinin", Chapter V of Bradykinin, Kallidin and Kallikrein, (Handbook of Expt. Pharmacol.), E. G. Erdoes (ed.), Vol 25, Springer Verlag (pub.), 1970, pp. 242-243. The inhibition potencies are determined on isolated guinea pig ileum, according to the commonly accepted manner described for antagonists of biologically active compounds in Schild, H.O., "pA, a New Scale for the Measurement of Drug Antagonism", Br. J. Pharmacol., Vol. 2, No. 3 (1947), pp. 189-206). In the assay, a dose-response curve is determined for the reference substance bradykinin. The dose of bradykinin which produces a half maximal contraction of tissue is the $ED_{50}$ dose. An amount of bradykinin equivalent to twice the $ED_{50}$ dose is administered to the tissue 30 seconds after the start of incubation of the tissue with a dose of antagonist. Doses of antagonist are increased in this protocol until pre-incubation with a dose of antagonist reduces the contraction in response to the double $ED_{50}$ dose of bradykinin to response of a single $ED_{50}$ dose of bradykinin. The pA2 value represents the negative logarithm of the molar concentration of antagonist which, in its presence, requires twice the amount of agonist to elicit the same response as when the agonist is given by itself. One unit of pA2 value represents an order of magnitude change in potency. For comparison, the negative log of the dose of bradykinin, the dose which causes half maximal contraction of the tissues, is commonly known as the pD2 value. The pD2 value for bradykinin is 7.4 on the guinea pig ileum.

### Test Method 2

### Plasma Extravasation in the Guinea Pig Trachea

Male Hartley guinea pigs weighing 450-700 g are anesthetized wth sodium pentobarbital. The right jugular vein is catherterized with a silastic tubing filled with 0.9% saline. The right carotid artery is cannulated. Evans blue dye is administered slowly via the jugular vein catheter. Five minutes later, bradykinin (0.4 ml/kg) and a test analogue (0.4 ml/kg) are co-administered intraarterially. Following an additional five minute period, the vasculature is perfused via the jugular vein with 0.9% saline. A 1 cm

section of tracheal tissue directly above the bifurcation is removed and weighed. Tissue is placed in tbes containing formamide. The tubes are placed in 50__C water-bath for 24 hours. Samples of the solvent from the tubes are transferred to cuvettes and measurements are taken on a fluorescence spectrophotometer. Calculations are made to determine dye content in the tissue. Data is expressed as ng dye/mg tissue.

## Test Method 3

### Mouse Abdominal Constriction Test

Compounds of the present invention are tested for analgesic activity using the mouse abdominal constriction assay. This assay is described in Hendershot, L.C., & J. Forsaith, "Antagonism of the Frequency of Phenylquinone-induced Writhing in the Mouse by Weak Analgesics and Nonanalgesics", J. Pharmacol., Vol. 125, (1959), pp. 237-240, and in Methods in Narcotics Research, S. Ehrenpreis and A. Neidle, (eds.), Marcel Dekker, Inc., New York (pub.), 1975, pp. 64-65, the disclosures of both these references being incorporated herein in their entirety by reference.

Male CF-1 mice (Charles River Breeding Laboratories, Inc.), weighing approximately 20 grams and food fasted overnight, are used in these assays. Test compounds are prepared for intraperitoneal (i.p.) injection, with compounds of the present invention being prepared in distilled deionized water so that the appropriate dose is given in 0.2 mls to a 20 gram mouse.

An i.p. injection of 0.02% phenylquinone (2.5 mg/kg) is given at a concentration of 0.25 ml/20 grams body weight. Five minutes later the mice receive an i.p. injection of test compound. Five minutes after the i.p. injection of test compound, the number of full body writhes are counted for the succeeding ten minutes. Percent analgesia in this assay is calculated as follows:

$$\frac{\text{Control number of writhes - Dosed number of writhes}}{\text{Control number of writhes}} \times 100$$

The mouse abdominal constriction assay was used to measure the antagonist activity of peptides of the present invention and related peptides as shown in Table 2:

Table 2

| Mouse Absominal Constriction Assay Results | | | |
|---|---|---|---|
| Compound No. | Peptide | Dose (mg/kg, I.P.) | Change from Control (%) |
| 1 | H-Arg-Pro-Hyp-Gly-Phe-Ser-DPhe-Phe-Arg-OH | 25 | -31 |
| 2 | H-Arg-Pro-Asn-Gly-Phe-Ser-DPhe-Phe-Arg-OH | 50 | 57 |
| 3 | H-Arg-Pro-Asn-DPro-Phe-Ser-DPhe-Phe-Arg-OH | 50 | 72,61 |
| 4 | H-Arg-Pro-Aap-Gly-Phe-Ser-DPhe-Phe-Arg-OH | 50 | 46 |

Compound 1 is a peptide disclosed in U.S. Patent No. 4,693,993. The negative result in this assay indicates that this peptide is a bradykinin agonist according to this assay. Compounds 2, 3 and 4 are peptides of the present invention. These compounds all show bradykinin antagonist activity in the assay.

## Test Method 4

## Cell Culture Method

### Background.

In many different tissues and isolated cell populations, bradykinin induces a receptor mediated rise in the concentration of cytosolic free calcium ($[Ca^{2+}]i$). In cultured NG108-15 neuronal cells (University of California at San Francisco, Cell Culture Facility, San Francisco, California), bradykinin increases $[Ca^{2+}]i$ in a dose dependent manner from a basal concentration of about 260 nM to approximately 1000 nM at a maximally effective concentration of bradykinin (1 X $10^{-6}$M). The half maximally effective concentration ($EC_{50}$) of bradykinin is 3 X $10^{-9}$M.

Bradykinin receptor antagonist potency is evaluated by determining the concentration of antagonist required to inhibit by 50% ($IC_{50}$) the increase in $[Ca^{2+}]i$ induced by 1 X $10^{-9}$M bradykinin (Bachem, Torrance, CA).

### NG108-15 Cell Culture.

NG108-15 cells are cultured in 75 $cm^3$ Corning flasks (Fisher Scientific, Cincinnati, OH) in Dulbecco's modified minimal essential medium (high glucose; Gibco, Grand Island, NY) containing 5% fetal bovine serum (Hyclone, Logan, UT) and HAT (hypoxanthine-aminopterin-thymidine, Sigma, St. Louis, MO).

### Measurement of Cytosolic Free Calcium Concentration in Cultured NG108-15 Cells

The concentration of cytosolic free $Ca^{2+}$ ($[Ca^{2+}]i$) is measured in suspensions of cultured NG108-15 cells loaded with the $Ca^{2+}$-sensitive fluorescent indicator fura-2 essentially as described by Osugi et al. (Journal Pharmacol. Exp. Ther., Vol. 240, (1987), pp. 617-622). Flasks of confluent cells are incubated for 60 minutes at 37° C in 20 mls of HEPES-buffered saline [140 mM NaCl, 5.4 mM KCl, 0.8 mM $MgSO_4$, 1.0 mM $CaCl_2$, 20 mM HEPES (N-2-. hydroxyethylpiperazine-N′-2-ethanesulfonic acid; Behring Diagnostics, LaJolla, CA), 10 mM glucose, 0.25% bovine serum albumin (Cat. #A-4503; Sigma Chemical Co., St. Louis, MO)] containing fura-2 acetoxymethyl ester (Molecular Probes, Inc., Eugene, OR) at a final concentration of 2 μM. The flasks are then washed twice with 30 mls of HEPES-buffered saline and the cells detached from the flasks in 15 mls of HEPES-buffered saline. The cell suspension is centrifuged at 25° C for 5 minutes at 350 x g and the cell pellet resuspended to a final volume of approximately 10 ml of HEPES-buffered saline/flask. The cell suspension is maintained at room temperature prior to use.

The fluorescence of the fura-2 loaded NG108-15 cells is measured at an excitation wavelength of 340 nM (10 nM slit) and an emission wavelength of 500 nM (10 nM slit) in a Perkin-Elmer LS-5 fluorescence spectrophotometer fitted with a magnetic stirrer and a thermostatted cell holder. An aliquot of the cell suspension (1.37 ml) is placed in a cylindrical glass cuvette (internal diameter of 8 mm) containing a teflon coated stir bar and warmed to 37° C in a water bath. After 5 minutes, the cuvette is placed in the cell holder of the fluorometer and stirring is initiated just prior to the addition of a test sample. Routinely, a test sample (0.014 ml of a 100-fold concentration solution) is added to the cuvette 30 seconds prior to the addition of 1 X $10^{-9}$ M bradykinin. Fluorescence is recorded continuously during the addition of the test sample and bradykinin.

Following completion of the fluorescence measurements, the cells are lysed with Triton X-100 at a final concentration of 10% (v/v) and the fluorescence of the $Ca^{2+}$ saturated fura-2 ($F_{max}$; fluorescence in the presence of 1 mM $Ca^{2+}$) and the $Mn^{2+}$-quenched fura-2 (autofluorescence (AF); fluorescence in the presence of 2 mM $Mn^{2+}$) determined. The $[Ca^{2+}]i$ before and after the addition of the test sample and/or bradykinin is determined from the observed fluorescence values ($F_{obs}$) using these values of $F_{max}$ and AF and the equations:

$F_{min} = AF + (F_{max}\text{-}AF/3.7$

$[Ca^{2+}]i = K_D(F_{obs}\text{-}F_{min})/(F_{max}\text{-}F_{obs}$

where $K_D$ is the dissociation constant for binding of $Ca^{2+}$ to the dye (224 nM; Grynkiewicz et al., J. Biol. Chem., Vol. 260, (1985), pp. 3440-3450).

To quantitate the extent of inhibition by an antagonist, the peak increase in $[Ca^{2+}]i$ from basal $[Ca^{2+}]i$ (i.e., $[Ca^{2+}]i$ just prior to the addition of bradykinin) is determined in the presence and absence of the

antagonist. The % inhibition is calculated as:

$$\frac{([Ca^{2+}]i \text{ increase in presence of test sample})}{([Ca^{2+}]i \text{ increase in absence of test sample})} \times 100$$

Agonistic activity is detected as an increase in fluorescence immediately following the addition of the test sample.

The cell culture method was used to measure the antagonist activity of peptides of the present invention and related peptides as shown in Table 3:

Table 3

| Cell Culture Method Results | | |
|---|---|---|
| Compound No. | Peptide | $IC_{50}$ |
| 1 | H-Arg-Pro-Hyp-Gly-Phe-Ser-DPhe-Phe-Arg-OH | Partial Agonist* |
| 2 | H-Arg-Pro-Asn-Gly-Phe-Ser-DPhe-Phe-Arg-OH | $5x10^{-7}$M |
| 5 | H-DArg-Arg-Pro-Hyp-Gly-Phe-Ser-DPhe-Phe-Arg-OH | $1x10^{-8}$M (10% agonist* |
| 6 | H-DArg-Arg-Pro-Asn-Gly-Phe-Ser-DPhe-Phe-Arg-OH | $2.2x10^{-8}$M |

* Compounds 1 and 5 are peptides disclosed in U.S. Patent No. 4,693,993. Compound 1 exhibits high agonist (bradykinin-like) activity such that an antagonist potency could not be calculated from this assay. Compound 5 also exhibits small amount of agonist activity.

Compounds 2 and 6 are peptides of the present invention and both exhibit only antagonist (no agonist) activity in this assay.

Another aspect of the present invention includes methods of producing anti-inflammatory activity and/or analgesia or treating rhinitis, rhinorhea and/or congestion in humans or lower animals by administering, to a human or lower animal in need of such present invention. This safe and effective amount can be given in a single dose or multiple doses repeatedly over the course of the treatment. Potential methods of administering the peptides of the present invention include per oral, parenteral (e.g., intravenous, intramuscular, intraperitoneal, subcutaneous), topical (epithelial, gingival, dermal or transdermal), intranasal, inhalation, sublingual, intravaginal, intrarectal, intrabuccal, or other internal administration means. Preferred methods of administering the peptides of this invention include dermal, transdermal, intranasal, and inhalation.

The phrase "safe and effective amount", as used herein, means an amount of a peptide or pharmaceutical composition of the present invention high enough to significantly positively modify the condition to be treated, but low enough to avoid serious side effects (at a reasonable benefit/risk ratio), within the scope of sound medical judgement. The safe and effective amount of the peptide or composition will vary with the particular condition being treated, the age and physical condition of the patient being treated, the severity of the condition, the duration of the treatment, the nature of concurrent therapy, the specific peptide or composition employed, the particular pharmaceutically-acceptable carrier utilized, and like factors within the knowledge and expertise of the attending physician.

The bradykinin antagonist peptides of this invention are useful for the treatment of one or more of the traumatic, inflammatory, pathological or normal conditions which are known to be medited by bradykinin or exacerbated by an overproduction of bradykinin. These conditions involve pain, inflammation, congestion and/or smooth muscle contractions. Such conditions include wounds, burns, rashes, snake bites, insect bites and stings, angina, arthritis, asthma, allergies, rhinitis, rhinorhea, sore throat pain, congestion, shock, inflammatory bowel disease, low blood pressure, systemic treatment of pain and inflammation, and/or low sperm motility which produces male infertility.

Therapeutic applications of the bradykinin antagonist peptides of the present invention include not only treatment for the production of bradykinin or related kinins by the animal but also the injection of bradykinin related peptides into an animal as a result of bites and stings. Topical application alone or in combination with subcutaneous utilization of the bradykinin antagonists of the invention can be employed to treat the effects of bradykinin-related peptides causing pain, inflammation, swelling and/or smooth muscle contraction.

It is estimated that the dosage range for topical applioation in such conditions as the pain and inflammation of wound, burns, rashes and sore throat would be from about 0.1 ml to about 500 mg per dose, preferably from about 1 mg to about 10 mg per dose. For a nasal or inhalation spray formulation suitable for treating rhinitis, allergies and asthma, a suitable dosage range would be from about 0.01 mg to about 100 mg per dose, preferably from about 0.5 mg to about 5 mg per dose. For an intravenous formulation suitable for the treatment of systemic inflammation, shock, arthritis, allergies, asthma and for increasing sperm motility, a suitable dosage range would be from about 0.1 mg/kg to about 500 mg/kg per dose, preferably from about 5 mg/kg to about 50 mg/kg per dose. For a per oral formulation for the treatment of inflammatory bowel disease or general pain and inflammation, a suitable dosage range would be from about 1 mg/kg to about 1000 mg/kg per dose, preferably from about 50 mg/kg to about 500 mg/kg per dose.

Another aspect of the present invention involves pharmaceutical compositions comprising a peptide of the present invention and a pharmaceutically-acceptable carrier. The peptides of the present invention typically comprise from about 0.1% to about 10% by weight of the pharmaceutical compositions of the present invention, preferably from about 0.5% to about 5%, and most preferably from about 1% to about 3%.

The term "pharmaceutically-acceptable carrier", as used herein, means one or more compatible solid or liquid filler diluents or encapsulating substances which are suitable for administration to a human or lower animal. The term "compatible", as used herein, means that the components of the pharmaceutical compositions are capable of being commingled with the compound of the present invention, and with each other, in a manner such that there is no interaction which would substantially reduce the pharmaceutical efficacy of the pharmaceutical composition under ordinary use situations. Pharmaceutically-acceptable carriers must, of course, be of sufficiently high purity and sufficiently low toxicity to render them suitable for administration to the human or lower animal being treated.

Some examples of substances which can serve as pharmaceutically-acceptable carriers are sugars such as lactose, glucose and sucrose; starches such as corn starch and potato starch; cellulose and its derivatives, such as sodium carboxymethylcellulose, ethylcellulose, cellulose acetate; powdered tragacanth; malt; gelatin; talc; stearic acid; magnesium stearate; calcium sulfate; vegetable oils such as peanut oil, cottonseed oil, sesame oil, olive oil, corn oil and oil of theobroma; polyols such as propylene glycol, glycerine, sorbitol, mannitol, and polyethylene glycol; sugar; alginic acid; pyrogen-free water; isotonic saline; phosphate buffer solutions; cocoa butter (suppository base); emulsifiers, such as the Tweens®; as well as other non-toxic compatible substances used in the pharmaceutical formulations. Wetting agents and lubricants such as sodium lauryl sulfate, as well as coloring agents, flavoring agents, excipients, tableting agents, stabilizers, antioxidants, and preservatives, can also be present.

While particular embodiments of the present invention have been described, it will be obvious to those skilled in the art that various changes and modifications to the peptides and compositions disclosed herein can be made without departing from the spirit and scope of the invention. It is intended to cover, in the appended claims, all such modifications that are within the scope of this invention.

## Claims

1. A bradykinin antagonist peptide characterized in that it has the structure:
H-L-Q-T-U-V-W-X-Y-Z-Arg-OH
wherein
(a) L is selected from nil, a single basic amino acid residue of the D- or L-configuration, a peptide containing from 2 to about 4 amino acid residues of the D-or L-configuration, at least one of which is a basic amino acid residue, and an N-terminal enzyme protecting group from the group consisting of acyl-type protecting groups, aromatic urethane-type protecting groups, and alkyl-type protecting groups;
(b) Q is selected from Arg, Lys and Orn;
(c) T and U are each independently selected from D- and L-cyclic amino acid residues, D- and L-aliphatic amino acid residues, L-aromatic amino acid residues, L-acidic amino acid residues, L-amide amino acid residues and L-hydroxamate amino acid residues; wherein at least one of T and U is selected from L-acidic amino acid residues, L-amide amino acid residues and L-hydroxamate amino acid residues;
(d) V is selected from aliphatic, cyclic and aromatic amino acid residues of the D- or L-configuration;
(e) W is selected from aliphatic and aromatic amino acid residues of the L-configuration;
(f) X is selected from aromatic and aliphatic amino acid residues of the D- or L-configuration;

(g) Y is selected from aromatic amino acid residues of the D-configuration;
(h) Z is selected from aromatic and aliphatic amino acid residues of the L-configuration;
and the pharmaceutically-acceptable salts thereof.

2. The peptide of Claim 1 characterized in that

(a) L is selected from nil, Arg, DArg, Lys, DLys, Lys-Lys, Met-Lys, Gly-Arg-Met-Lys, DLys-Lys, Orn, Har, DHar, 5-guanidinopentanoyl, 6-guanidinohexanoyl, 6-aminohexanoyl and 5-aminopentanoyl;

(b) Q is selected from Arg, Lys, and Orn;

(c) T and U are each independently selected from Pro, DPro, Hyp, Azt, Thz, Inp, Epr, Asp, Glu, Asn, Gln, Cpr, Cmp, Aap, Aor, Nha and Nhg; wherein at least one of T and U is selected from Asp, Glu, Asn, Gln, Cpr, Cmp, Aap, Aor, Nha and Nhg;

(d) V is selected from Gly, Ala, DPro, DHyp and Sar;

(e) W is selected from Phe, Thi, Nap, Pyr, Nph, Cph, Mty and Tyr;

(f) X is selected from Ser, Thr, Ala, Gly, DPhe, DThi, DNap, DPyr and DCph;

(g) Y is selected from DPhe, DTyr, DThi, DNap, DPyr, DNph, DCph, DMty, DTrp, DHis, DHph and DPgl;

(h) Z is selected from Phe, Tyr, Thi, Pyr, Nap, Mty, Nph and Cph.

3. The peptide of Claim 2 wherein

(a) L is selected from DArg, Lys-Lys, and nil; preferably L is DArg;

(b) Q is Arg;

(c) T and U are each independently selected from Pro, Hyp, Asp, Glu, Asn, Gln, Cpr, Cmp, Aap, Aor, Nha and Nhg; wherein at least one of T and U is selected from Asp, Glu, Asn, Gln, Cpr, Cmp, Aap, Aor, Nha and Nhg;

(d) V is selected from DPro, DHyp, Gly and Ala; preferably V is Gly or DPro;

(e) W is selected from Phe and Tyr; preferably W is Phe;

(f) X is selected from Ser, Thr, Ala and Gly; preferably X is Ser;

(g) Y is selected from DPhe, DTyr, DTrp and DHis; preferably Y is DPhe; and

(h) Z is selected from Phe and Tyr; preferably Z is Phe.

4. The peptide of any of Claims 1-3 wherein one of T or U, preferably T, is selected from Pro and Hyp; and the other of T or U, preferably U, is selected from Asp, Glu, Asn, Gln Cpr, Cmp, Aap, Aor, Nha and Nhg, preferably from Asp, Glu, Aap, Asn and Gln, most preferably from Aap, Asn and Gln.

5. The peptide of Claim 2 characterized in that it has a structure wherein:

(a) Q is Arg;
(b) T is Pro or Hyp;
(c) U is Aap, Asn or Gln;
(d) V is Gly or DPro;
(e) W is Phe;
(f) X is Ser;
(g) Y is DPhe;
(h) Z is Phe; and
(i) L is DArg.

6. A peptide characterized in that it has a structure selected from:

H-Arg-Pro-Asn-DPro-Phe-Ser-DPhe-Phe-Arg-OH,
H-Arg-Pro-Asn-Gly-Phe-Ser-DPhe-Phe-Arg-OH,
H-DArg-Arg-Pro-Asn-DPro-Phe-Ser-DPhe-Phe-Arg-OH,
H-DArg-Arg-Pro-Asn-Gly-Phe-Ser-DPhe-Phe-Arg-OH,
H-Lys-Lys-Arg-Pro-Asn-DPro-Phe-Ser-DPhe-Phe-Arg-OH,
H-Lys-Lys-Arg-Pro-Asn-Gly-Phe-Ser-DPhe-Phe-Arg-OH,
H-Arg-Pro-Aap-DPro-Phe-Ser-DPhe-Phe-Arg-OH,
H-Arg-Pro-Aap-Gly-Phe-Ser-DPhe-Phe-Arg-OH,
H-DArg-Arg-Pro-Aap-DPro-Phe-Ser-DPhe-Phe-Arg-OH,
H-DArg-Arg-Pro-Aap-Gly-Phe-Ser-DPhe-Phe-Arg-OH,
H-Lys-Lys-Arg-Pro-Aap-DPro-Phe-Ser-DPhe-Phe-Arg-OH and
H-Lys-Lys-Arg-Pro-Aap-Gly-Phe-Ser-DPhe-Phe-Arg-OH.

7. A pharmaceutical composition characterized in that it comprises the peptide of any of Claims 1-6 and a pharmaceutical carrier.

8. The use of a safe and effective amount of the peptide of any of Claims 1-6 for the manufacture of a medicament to treat pain, inflammation, congestion or smooth muscle contraction.